# EUROPÄISCHE PATENTANMELDUNG

(11) **EP 1 136 824 A1**
(43) Veröffentlichungstag der Anmeldung: **26.09.2001**
(21) Anmeldenummer: 00105817.1
(22) Anmeldetag: 18.03.2000
(51) Int. Cl.: G01N 33/96

(54) **Flüssigkeit zur Nachbildung des rheologischen Verhaltens von Bioflüssigkeiten**

(71) Anmelder: proRheo GmbH, 75382 Althengstett (DE)
(72) Erfinder: Nelson, Karen, 60590 Frankfurt (DE)
(74) Vertreter: Klocke, Peter, Dipl.-Ing.

(57) **Zusammenfassung**

Flüssigkeit zur Nachbildung des rheologischen Verhaltens von Bioflüssigkeiten, insbesondere Blut, bestehend aus einer Suspension aus Mikro-Kunststoffpartikeln und Wasser, dem mindestens ein Mittel, das die Mikrokunststoffpartikel in Suspension hält, eine viskoelastische Flüssigkeit zur Einstellung der nicht newtonschen Viskosität, und ein Salz zur Einstellung der physiologischen Elektrolytkonzentration beigefügt ist. Diese Kalibrierflüssigkeit ist geeignet, als physikalisches Blutäquivalent eingesetzt zu werden, das auf die Grundnormale der Physikalisch-Technischen Bundesanstalt (PTB) rückführbar ist. Sie erfüllt alle Anforderungen an eine Kalibrier- und Eichsubstanz und kann als langzeitstabile Messprobe in ausreichender Menge und ausreichender Genauigkeit reproduzierbar hergestellt werden. Es ist damit möglich, Geräte und Komponenten in vitro normgerecht zu testen und zu prüfen.

## Beschreibung

Die vorliegende Erfindung betrifft eine Flüssigkeit zur Nachbildung des rheologischen Verhaltens von Bioflüssigkeiten, insbesondere Blut.

Zur Rheologieforschung, wie beispielsweise der Untersuchung des Fließverhaltens einer Bioflüssigkeit, insbesondere Blut, in operativ veränderten Gefäßen, der Verschleißprüfung von Herzklappenprothesen, Qualitätsprüfung von Herzpumpen, Untersuchung von Kreislaufmodellen, Eichung und Kalibrierung von Rheometern, u. a. ist es erforderlich, dies mit einer Flüssigkeit durchzuführen, die hinsichtlich des rheologischen Verhaltens möglichst gleiche Eigenschaften aufweist wie die jeweilige Bioflüssigkeit. Blut ist jedoch eine Suspension, bestehend aus einer Trägerflüssigkeit und einem Anteil aus flexiblen Festkörpern, den man als Hämatokritanteil im Blut angibt. So entspricht ein Hämatokritanteil von 42, z. B., 42 % Feststoffteilchen im Vollblut. Außerhalb des menschlichen Körpers ist Humanblut nur bedingt haltbar und reagiert sofort mit der Umgebung (Gerinnung oder Sedimentierung). Infolgedessen ist eine vom Mensch abgenommene Blutprobe durch diese Reaktion auf die Umwelteinflüsse nur sehr kurzeitig mit Blut im menschlichen Körper zu vergleichen. Sind größere Mengen von Humanblut zu Testzwecken notwendig, so kann nicht mit entnommenem Blut gearbeitet werden. Auch die Verwendung von Blutkonserven ist nicht möglich, da diese Zusatzstoffe enthalten, welche die viskoelastische Eigenschaften beeinflussen. Das Humanblut verschiedener Probanden ähnelt sich in seinem rheologischen Verhalten, wobei die Messwertstreuung jedoch sehr groß ist. Eine echte Humanblutprobe kann daher nicht verwendet werden, um Geräte oder ähnliche Teile für den Einsatz im Blutkreislauf, zum Bluttransport oder auch zur Justierung und Kalibrierung von Blutmessgeräten zu prüfen.

Aus diesem Grunde wurde bereits versucht, eine Flüssigkeit mit Glaskugeln zu versehen, wobei diese jedoch aufgrund ihres Gewichts nicht in der Schwebe gehalten werden können. Aufgrund dessen, dass Blutteilchen, trotz ihrer bereits geringen Größe (rote Blutzellen 7 bis 8 µm, weiße Blutzellen 9 bis 12 µm) sich durch Kapillare mit noch geringerem Durchmesser hindurch bewegen und sich dabei verformen, sind sie rheologisch wie ein Feststoffteilchen zu behandeln. Ein Gerät, das nur mit einer Flüssigkeit kalibriert wird, reagiert bei der Anwendung mit verformbaren Feststoffkörpern also beispielsweise im Körper, anders, so dass hier Schwierigkeiten zu erwarten sind.

Bis heute gibt es keinen Standard, der wirklich einer Bioflüssigkeit, wie beispielsweise Blut entspricht, d. h. ein Standard, der aus zwei Teilen besteht, nämlich aus einer Flüssigkeit und (verformbaren) Feststoffkörper und sich rheologisch wie die wirkliche Flüssigkeit verhält.

Teilweise wurde in der Praxis auch Blutplasma verwendet. Dies hat jedoch ebenfalls wie andere bis dahin verwendete Flüssigkeiten keine Feststoffkörper und liefert daher nur eine Teilinformation.

Es besteht ein enormer Bedarf für eine Kalibrierflüssigkeit, um beispielsweise Labormessgeräte zu eichen, damit immer wieder reproduzierbare Vergleichsmessungen durchgeführt werden können. Dies ist besonders dann wichtig, wenn Medikamenteneinstellungen aufgrund der rheologischen Eigenschaften des Blutes erfolgen und hierzu Blutmessungen an verschiedenen Stellen, beispielsweise in der Klinik und später beim behandelnden Arzt durchgeführt werden. Es besteht dann keine Übereinstimmung der Messungen innerhalb und außerhalb der Klinik.

Der vorliegenden Erfindung liegt daher die Aufgabe zugrunde, eine blutähnliche Suspension zu entwickeln, die anwender- und geräteunabhängig als Normflüssigkeit für Bioflüssigkeiten, insbesondere Blut, geeignet ist.

Diese Aufgabe wird erfindungsgemäß durch eine Kalibrierflüssigkeit mit den Merkmalen des Anspruchs 1 gelöst. Weitere vorteilhafte Ausgestaltungen sind den Unteransprüchen zu entnehmen.

Die Kalibrierflüssigkeit gemäß der Erfindung besteht aus einer Suspension aus Mikro-Kunststoffpartikeln und einer Flüssigkeit. Die Mikro-Kunststoffpartikel können elastisch sein. Ihre Größe entspricht im wesentlichen der Größe der Blutkörperchen, d. h. sie liegen im Bereich von 5 bis 10 µm. Die Mikro-Kunststoffpartikel dienen einerseits dazu, als partikulärer Anteil die Blutkörperchen zu imitieren und darüber hinaus eine physiologische oder pathologische Blutviskosität zumindest im newtonschen Bereich (Scherrate > 200 s⁻¹) einzustellen. Als Mikrokunststoffpartikel können hierzu beispielsweise Kügelchen aus Polyamid mit einem Durchmesser von 5 bis 10 µm verwendet werden.

Die Flüssigkeit als nicht partikulärer Anteil zur Imitation des Plasmas besteht aus Wasser, dem mindestens ein Mittel, das die Mikro-Kunststoffpartikel in Suspension hält, eine viskoelastische Flüssigkeit zur Einstellung der nichtnewtonschen Viskosität, und ein Salz zur Einstellung der physiologischen Elektrolytkonzentration beigefügt ist. Als Suspensions-Mittel sind insbesondere Emulgatoren, vorzugsweise Lecithin geeignet. Die Konzentration des Emulgators ist abhängig von der Menge der Mikro-Kunststoffpartikel und steigt mit dieser Menge. Dieses Mittel ändert selbstverständlich auch die nichtnewtonschen Eigenschaften der Kalibrierflüssigkeit. Als viskoelastische Flüssigkeit sind beispielsweise Tensidlösungen, vorzugsweise jedoch Polyethylenglykole, bevorzugt.

Als Salz zur Einstellung der physiologischen oder pathophysiologischen Elektrolytkonzentrationen werden Natriumchlorid und/oder andere Salze zugesetzt.

Gemäß weiteren bevorzugten Ausbildungen wird zur Vermeidung von Bakterien-und Pilzwachstum ein Konservierungsmittel beigefügt. Hierbei hat sich Kaliumsorbat als geeignet erwiesen. Auch andere gleichwirkende Konservierungsmittel sind geeignet.

Da die Kalibrierflüssigkeit auch ihre Eigenschaften hinsichtlich der rheologischen Haltbarkeit zumindest über einen gewissen Zeitraum beibehalten soll, hat sich gezeigt, dass durch die Zugabe eines Dispergators erfolgreich ein Zusammenkleben der Mikro-Kunststoffpartikel verhindert werden kann. Damit wird erreicht, dass die Viskosität über einen gewissen Zeitraum von ca. 1 Monat konstant gehalten werden kann und nicht wie bei der Abwesenheit eines Dispergators bereits in diesem Zeitraum zunimmt. Mit dem vorstehend erwähnten Konservierungsmittel konnte lediglich ein Wachstum von Bakterien oder Pilzen, nicht jedoch ein Verklumpen der Mikro-Kunststoffpartikel verhindert werden.

Die erfindungsgemäß ausgebildete Kalibrierfüssigkeit erfüllt alle Anforderungen an eine langzeitstabile Messprobe. Sie kann in ausreichender Menge und ausreichender Genauigkeit reproduzierbar hergestellt werden. Mit ihr ist es möglich, Geräte und Komponenten in vitro normgerecht zu testen und zu prüfen. Sie ist allerdings nicht geeignet, in vivo eingesetzt zu werden, da sie die Anforderung eines Blutersatzstoffes zur Aufrechterhaltung der Vitalfunktion des Menschen nicht erfüllt.

Bei einem Ausführungsbeispiel wurden Mikro-Kunststoffpartikel aus Nylon in einer Größe von 5 µm in einer Konzentration von ca. 23 % Gewicht/Volumen eingesetzt. Damit wurde eine normale Blutviskosität im newtonschen Bereich mit einer Scherrate > 200s⁻¹ eingestellt. Als nicht partikuläre Anteile wurden 1,15 % Lecithin, 0,25 % Polyethylenglykol, 0,9 % Natriumchlorid und 0,03 % Kaliumsorbat eingesetzt. Alle Konzentrationen sind in Gewicht/Volumen angegeben. Diese nicht partikulären Anteile werden gewogen und mit der entsprechenden Menge destilliertem Wasser in einem Mixer gemischt. Die Lösung wird anschließend zur Einstellung des entsprechenden Endvolumens entgast. Die Nylonpartikel werden gewogen und mit der entsprechenden Menge des Plasmaanteils mit den nicht partikulären Anteilen im Mixer gemischt. Diese Lösung wird anschließend entgast, um das Endvolumen einzustellen.

Zur Erhöhung der Haltbarkeit in rheologischer Hinsicht wurden 40 bis 100 µl vorzugsweise 50 bis 55 µl eines Dispergators beigefügt. Dieser besteht aus einem Natriumsalz einer niedrigmolekularen Polyacrylsäure, und wird beispielsweise unter dem Handelsname Busperse der Firma Buckman Laboratories vertrieben.

Diese hergestellte Suspension besteht wie Blut aus nicht partikulären und partikulären Anteilen. Im niedrigen Schergeschwindigkeitsbereich von 0,05 x 10⁻³ bis 120 s⁻¹ zeigt die Suspension das typische Verhalten einer nichtnewtonschen Suspension, äquivalent zu Humanblut. Im höheren Bereich von 120 bis 300 s⁻¹ zeigt sie das geforderte newtonsche Verhalten. Die Testsubstanz besteht aus Partikeln, die in einer Flüssigkeit suspendiert sind. Die Viskosität und der Feststoffanteil sind einstellbar.

Die Mikro-Kunststoffpartikel können auch hinsichtlich des Durchmessers variiert werden, so dass Mikrokügelchen mit verschiedenen Durchmessern in der Kalibrierflüssigkeit enthalten sind.

## Patentansprüche

1. Flüssigkeit zur Nachbildung des rheologischen Verhaltens von Bioflüssigkeiten, insbesondere Blut, bestehend aus einer Suspension aus Mikro-Kunststoffpartikeln und Wasser, dem mindestens
ein Mittel, das die Mikrokunststoffpartikel in Suspension hält,
eine viskoelastische Flüssigkeit zur Einstellung der nicht newtonschen Viskosität, und
ein Salz zur Einstellung der physiologischen Elektrolytkonzentration beigefügt ist.

2. Fllüssigkeit nach Anspruch 1, **dadurch gekennzeichnet, dass** die Kunststoffpartikel als Kügelchen aus Polyamid mit einem Durchmesser von 5 bis 20 µm, vorzugsweise 5 bis 10 µm ausgebildet sind.

3. Flüssigkeit nach Anspruch 1 oder 2, **dadurch gekennzeichnet, dass** das Mittel zum Halten der Mikro-Kunststoffpartikel in Suspension ein Emulgator, vorzugsweise Lecithin, ist.

4. Flüssigkeit nach einem der vorangegangenen Ansprüche, **dadurch gekennzeichnet, dass** die viskoelastische Flüssigkeit eine Tensidlösung, ist.

5. Flüssigkeit nach einem der vorangegangenen Ansprüche 1 bis 3, **dadurch gekennzeichnet, dass** die viskoelastische Flüssigkeit Polyethylenglykol ist.

6. Flüssigkeit nach einem der vorangegangenen Ansprüche, **dadurch gekennzeichnet, dass** ein Konservierungsmittel zur Vermeidung von Bakterien- und Pilzwachstum beigefügt ist.

7. Flüssigkeit nach einem der vorangegangenen Ansprüche, **dadurch gekennzeichnet, dass** ein Dispergator zur Haltbarmachung hinsichtlich der rheologischen Eigenschaften beigefügt ist.

8. Verwendung einer Flüssigkeit bestehend aus einer Suspension aus Mikro-Kunststoffpartikeln und Wasser, dem mindestens ein Mittel, das die Mikrokunststoffpartikel in Suspension hält, eine viskoelastische Flüssigkeit zur Einstellung der nicht newtonschen Viskosität, und ein Salz zur Einstellung der physiologischen Elektrolytkonzentration beigefügt ist, zur Nachbildung des rheologischen Verhaltens von Bioflüssigkeiten, insbesondere Blut.
